# EUROPEAN PATENT APPLICATION

(11) **EP 3 284 822 A1**
(43) Date of publication of application: **21.02.2018**
(21) Application number: 16780157.0
(22) Date of filing: 15.04.2016
(51) Int. Cl.: C12N 15/09, A61K 9/127, A61K 31/7125, A61K 48/00, A61P 27/02, C12N 15/113

(54) **THERAPEUTIC AGENT FOR EYE DISEASE**

(30) Priority: 17.04.2015 JP 2015085470
(71) Applicant: The University of Tokyo, Tokyo 113-8654 (JP); Tokyo Medical University, Shinjuku-ku, Tokyo 160-8402 (JP); Takeuchi, Hirofumi, Gifu-shi, Gifu 501-1196 (JP); Bonac Corporation, Kurume-shi, Fukuoka 839-0861 (JP)
(72) Inventor: TAKEUCHI, Hirofumi, Gifu-shi Gifu 501-1196 (JP); USUI, Tomohiko, Tokyo 113-8654 (JP); KURODA, Masahiko, Tokyo 160-8402 (JP); TAKANASHI, Masakatsu, Tokyo 160-8402 (JP); OHNO, Shinichiro, Tokyo 160-8402 (JP); TOYOFUKU, Hidekazu, Kurume-shi Fukuoka 839-0861 (JP); TAHARA, Kohei, Gifu-shi Gifu 501-1196 (JP); ONODERA, Risako, Gifu-shi Gifu 501-1196 (JP)
(74) Representative: J A Kemp
(86) International application number: PCT/JP2016/062183
(87) International publication number: WO 2016/167366

(57) **Abstract**

The present invention provides a single stranded nucleic acid molecule represented by the following formula (I):
5'-uagcaccauuugaaaucaguguucc-P-ggaacacugauuucaaauggugcuauu-3' (I)
(SEQ ID NO:1)
wherein -P- shows a proline derivative linker represented by the following formula (Ia): 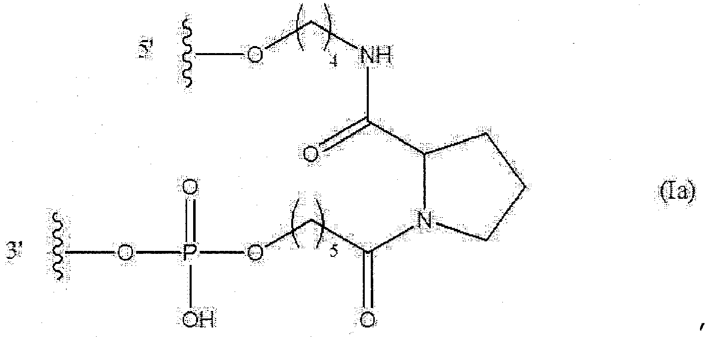 and
an agent for treating corneal diseases such as ocular surface disorders and the like, comprising the single stranded nucleic acid molecule as an active ingredient.

SEQUENCE LISTING

<110>  The University of Tokyo
       Tokyo Medical University
       TAKEUCHI, Hirofumi
       BONAC CORPORATION

<120>  Agent for treating ocular diseases

<130>  092439

<150>  JP 2015-085470
<151>  2015-04-17

<160>  4

<170>  PatentIn version 3.5

<210>  1
<211>  52
<212>  RNA
<213>  Artificial Sequence

<220>
<223>  modified miR-29b


<220>
<221>  misc_feature
<222>  (25)..(26)
<223>  These nucleotides are linked via a proline derivative linker.

<400>  1
uagcaccauu ugaaaucagu guuccggaac acugauuuca aauggugcua uu          52


<210>  2
<211>  25
<212>  RNA
<213>  Artificial Sequence

<220>
<223>  guide strand of modified miR-29b

<400>  2
uagcaccauu ugaaaucagu guucc                                        25


<210>  3
<211>  27
<212>  RNA
<213>  Artificial Sequence

<220>
<223>  passenger strand of modified miR-29b

<400>  3
ggaacacuga uuucaaaugg ugcuauu                                      27


<210>  4
<211>  48
<212>  RNA

<213>  Artificial Sequence

<220>
<223>  control PshRNA



<220>
<221>  misc_feature
<222>  (23)..(24)
<223>  These nucleotides are linked via a proline derivative linker.

<400>  4
uacuauucga cacgcgaagu uccggaacuu cgcgugucga auaguauu                    48

## Description

### [Technical Field]

The present invention relates to a novel single stranded nucleic acid molecule and use thereof for treating eye diseases. More particularly, the present invention relates to a nucleic acid molecule, which is a miR-29b derivative, and an agent comprising same for treating corneal diseases including ocular surface disorders.

### [Background Art]

While cornea is an innately avascular transparent tissue, it loses transparency due to scaring or angiogenesis therein by various kinds of inflammation, edema and the like. Stevens-Johnson syndrome (SJS), ocular cicatricial pemphigoid (OCP), thermal and chemical burns (alkali burn, acid burn, thermal burn etc.) and the like are typical examples of such opacity of cornea. In these diseases, a corneal limbus region including corneal epithelial stem cells is extensively damaged, which results in corneal angiogenesis and corneal cicatricial opacity, and severe dysfunction of visual performance. Such diseases that cause exhaustion of corneal epithelial stem cells are referred to as "refractory ocular surface disorders", and cannot be sufficiently treated by a conventional drug therapy. Also, it has been a clinical problem that various corneal inflammations, hypoxia due to contact lens, infectious corneal disease and corneal edema due to corneal endothelial disorder cause cicatricial opacity and angiogenesis, which reduces corneal transparency. Therefore, development of a drug capable of treating opacity of cornea has been desired.

The progress in biotechnology in recent years has revealed various nucleic acids that exert a physiologically active function within a cell. For example, a micro RNA (miRNA), which is an endogenous non-coding RNA consisting of about 20 to 25 bases encoded in a genome, is known to cause inhibition of translation from or degradation of mRNA of a target gene present in a cell, thereby controlling the expression of the target gene. At first, miRNA is transcribed as a primary transcript consisting of hundreds to thousands of bases (Primary miRNA (Pri-miRNA)) from a miRNA gene in a genome DNA, and nextly, it undergoes processing to generate a precursor miRNA (pre-miRNA) consisting of about 60 to 70 bases and having a hairpin structure. Then, the pre-miRNA is translocated from nucleus to cytosol, undergoes further processing by an RNase called Dicer to generate a double stranded mature miRNA consisting of about 20 to 25 bases. One strand (guide strand) of the double stranded mature miRNA is known to form a complex with proteins called RISC and act on mRNA of the target gene, thereby inhibiting the expression of the target gene (e.g., see non-patent document 1).

One thousand or more kinds of miRNAs are known in human, mouse and the like, each of which is suggested to control the expression of a plurality of target genes and thereby involved in various life phenomena such as proliferation and differentiation of a cell. For example, miRNAs involved in differentiation of hematopoietic cells or neurons have been reported (e.g., see non-patent document 2). Since controlling the expression/function of a plurality of target genes by an miRNA is useful to relieve or treat a disease symptom caused by abnormal expression of a certain gene or group of genes, miRNA is expected to be developed as a therapeutic drug.

### [Document List]

### [non-patent documents]

non-patent document 1: Elbashir SM et. al. Nature 411:494-498 (2001)
non-patent document 2: Science 303: 654 83-86 (2004)

### [SUMMARY OF THE INVENTION]

### [Problems to be Solved by the Invention]

An object of the present invention is to provide a pharmaceutical using a nucleic acid molecule effective for the treatment of eye diseases.

### [Means of Solving the Problems]

The present inventors have conducted intensive studies in an attempt to achieve the above-mentioned object and focused on miR-29b. The present inventors constructed a single stranded nucleic acid molecule (single stranded artificially matched miR-29b) wherein ends of a double stranded RNA containing a duplex miRNA consisting of the guide strand of miR-29b and a passenger strand completely complementary thereto (artificially matched miRNA) are linked to each other with a proline derivative linker, encapsulated the single stranded nucleic acid molecule into a liposome and instilled same in the eye of a mouse alkaline burn model. As a result, the present inventors found that angiogenesis and scarring were remarkably suppressed.

Based on these findings, the present inventors have concluded that a replacement therapy by instillation of a single stranded artificially matched miR-29b is useful to treat corneal diseases accompanied by corneal angiogenesis or cicatricial opacity and the like including ocular surface disorders, which resulted in the completion of the present invention.

Accordingly, the present invention relates to the following.
[1] A single stranded nucleic acid molecule represented by the following formula (I):
   5'-uagcaccauuugaaaucaguguucc-P-ggaacacugauuucaaauggugcuauu-3' (I)
      (SEQ ID NO:1),
   wherein -P- shows a proline derivative linker represented by the following formula (Ia): 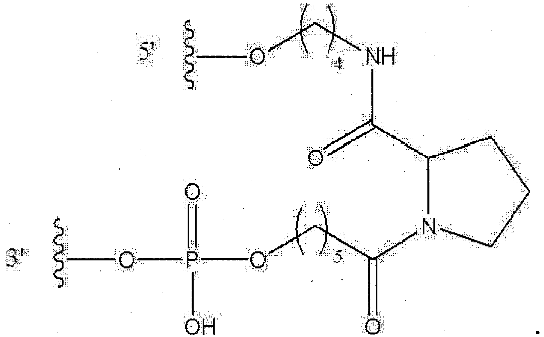
[2] An agent for treating a corneal disease comprising the single stranded nucleic acid molecule according to [1] as an active ingredient.
[3] The agent according to [2], wherein the corneal disease is accompanied by corneal angiogenesis or cicatricial opacity.
[4] The agent according to [3], wherein the corneal disease is selected from the group consisting of an ocular surface disorder, corneal inflammation, hypoxia due to contact lens, an infectious corneal disease, corneal opacity due to reduced function of corneal endothelium and gelatinous drop-like corneal dystrophy.
[5] The agent according to [3], wherein the corneal disease is a refractory ocular surface disorder selected from Stevens-Johnson syndrome, ocular cicatricial pemphigoid, and thermal and chemical burns.
[6] The agent according to any of [2] to [5], wherein the single stranded nucleic acid molecule is encapsulated in a liposome.
[7] The agent according to any of [2] to [6], which is an eye drop.
[8] A method of treating a corneal disease, comprising administering to a subject in need thereof a therapeutically effective amount of the single stranded nucleic acid molecule according to [1].
[9] The stranded nucleic acid molecule according to [1] for use in the treatment of a corneal disease.
[10] Use of the single stranded nucleic acid molecule according to [1] for the manufacture of an agent for treating a corneal disease.

### [Effect of the Invention]

The present invention makes it possible to treat corneal diseases including refractory ocular surface disorders.

### [Brief Description of the Drawings]

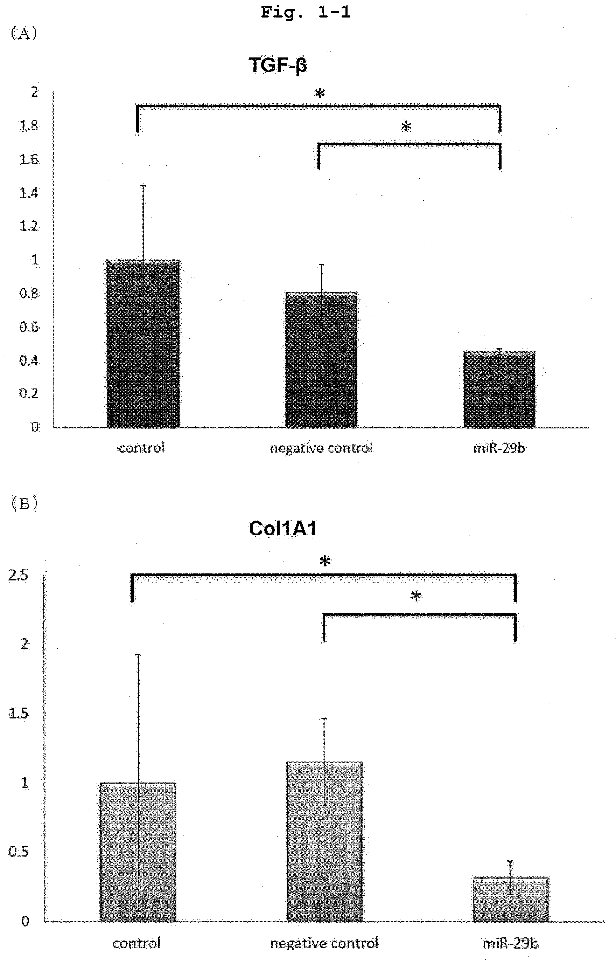

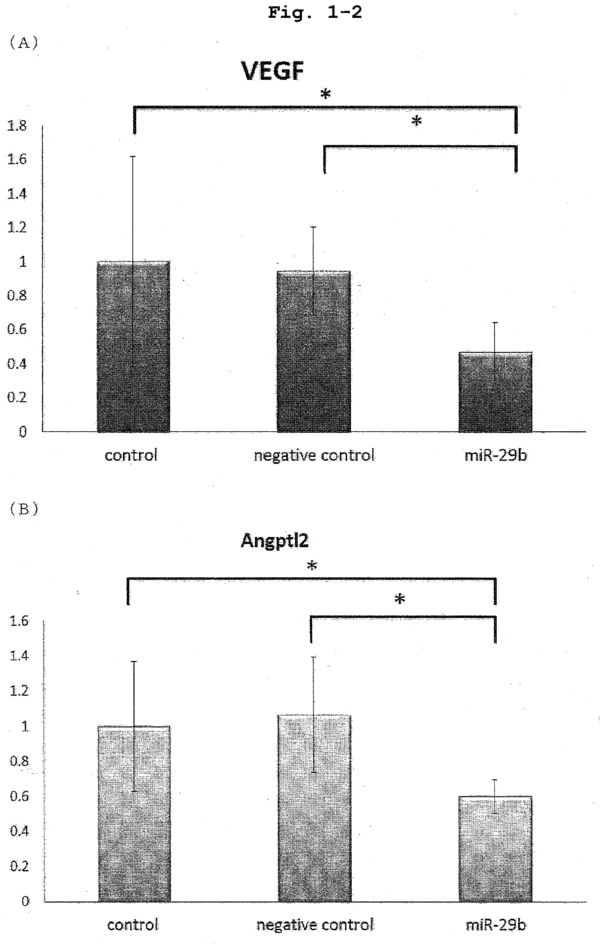

[Fig. 1-1] Fig. 1-1 shows suppression of the expression of TGF-β (A) and Col1A1 (B) in cultured corneal parenchymal cells by addition of miR29b-PshRNA. The vertical axis shows a relative expression level when the expression level in the control (non-addition group) is defined as 1. Negative control shows a group to which a non-specific PshRNA was added. miR-29b shows a group to which miR29b-PshRNA was added. *: p<0.01
[Fig. 1-2] Fig. 1-2 shows suppression of the expression of VEGF (A) and Angptl2 (B) in cultured corneal parenchymal cells by addition of miR29b-PshRNA. The vertical axis shows a relative expression level when the expression level in the control (non-addition group) is defined as 1. Negative control shows a group to which a non-specific PshRNA was added. miR-29b shows a group to which miR29b-PshRNA was added. *: p<0.01

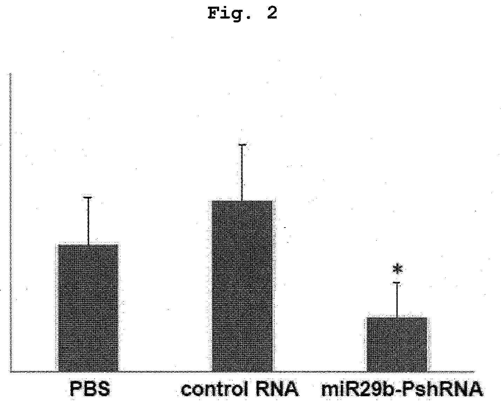

[Fig. 2] Fig. 2 shows suppressive effects of instillation of mir29b liposome-PshRNA on angiogenesis and scarring in a mouse alkaline burn model. The vertical axis shows percentage of blood vessel area per unit corneal area. PBS shows a group to which PBS was instilled, control RNA shows a group to which a liposome preparation of a non-specific PshRNA was instilled, miR29b-PshRNA shows a group to which a liposome preparation of miR29b-PshRNA was instilled. *: p<0.01

### [Description of Embodiments]

Hereinafter, the present invention is explained in detail.

### 1. Single stranded artificially matched miR-29b (the nucleic acid of the present invention)

The single stranded artificially matched miR-29b, which is the therapeutic drug for ocular surface disorders of the present invention, is a single stranded nucleic acid molecule represented by the following formula (I):
5'-UAGCACCAUUUGAAAUCAGUGUUCC-P-GGaacacugauuucaaauggugcuaUU-3' (I)
   (SEQ ID NO:1)
wherein -P- shows a proline derivative linker represented by the following formula (Ia):

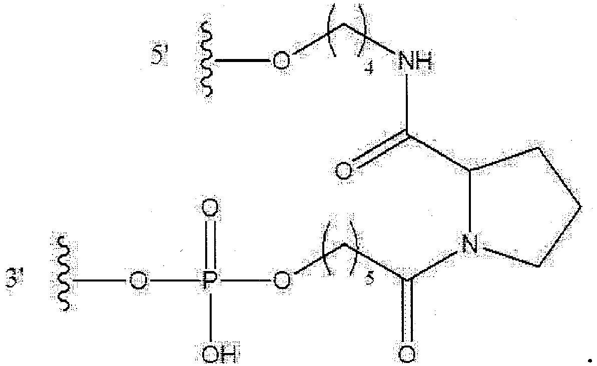

In the formula (I), the underline shows the guide strand sequence (namely, the 3' side mature miRNA sequence (mmu-mir-29b-3p; miRBase Accession No. MIMAT0000127) (the bold underline in the formula (II)) of the pre-miRNA of naturally occurring mouse miR-29b1 (mmu-mir-29-1; miRBase Accession No. MI0000143) (the formula (II)):

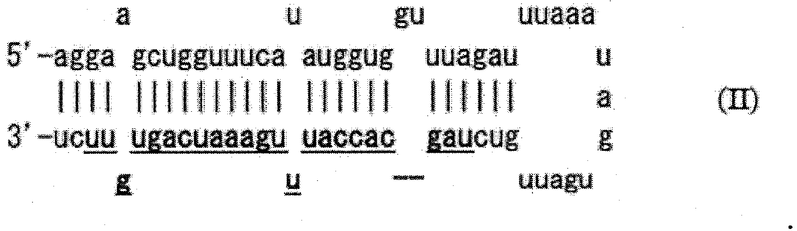

In the formula (I), lower cases show the passenger strand sequence completely complementary to the guide strand sequence. The guide strand sequence comprises an additional sequence consisting of 2 nucleotides (CC) at the 3'-end, the passenger strand has a sequence complementary to the additional sequence (GG) at the 5'-end. As a result, a completely complementary intramolecular duplex structure is formed between the guide strand sequence and the additional sequence and the passenger strand sequence and the sequence complementary to the additional sequence. The passenger strand has an overhang consisting of 2 nucleotides (UU) at the 3'-end.

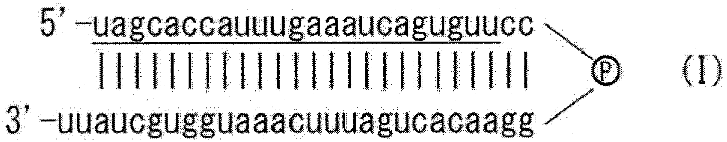

Since the nucleic acid of the present invention can suppress an innate immune response by a Toll-like receptor (TLR) by making naturally occurring miR-29b a single stranded molecule while maintaining its target specificity, side effects can be reduced when it is administered to an animal. It is considered to act in a Dicer-independent manner, by forming a characteristic duplex structure via a non-nucleotide linker. In addition, since its *in vivo* stability is increased by forming the duplex structure, it shows preferable pharmacokinetics without using modified nucleotides and can be prevented from reduction of activity and the like due to modification. Furthermore, it becomes a more stable structure compared to naturally occurring miR-29b by eliminating mismatches.

In the present invention, nucleic acid is RNA, chimeric nucleic acid of RNA and DNA (hereinafter to be referred to as chimeric nucleic acid) or hybrid nucleic acid. As used herein, chimeric nucleic acid means that a single stranded or double stranded nucleic acid contains RNA and DNA in one strand of nucleic acid, and hybrid nucleic acid refers to a double stranded nucleic acid having RNA or chimeric nucleic acid for one strand and DNA or chimeric nucleic acid for the other strand.

The nucleic acid of the present invention may be a free form or in the form of a salt.

Since a natural nucleic acid is easily degraded by nucleases present in a cell, the nucleic acid of the present invention may be modified to be a modified product resistant to various degrading enzymes by modifying a part or all of the constituent nucleotides. The modified product of the present invention includes, but are not limited to, those wherein the sugar chain moiety is modified (e.g., 2'-O methylation, 2'-fluoration), those wherein the base moiety is modified, and those wherein the phosphate moiety and hydroxyl moiety are modified (e.g., biotin, amino group, lower alkylamine group, acetyl group etc.). However, as mentioned above, since the nucleic acid of the present invention has an improved in vivo stability compared to a normal double stranded RNA due to its structural property, that wherein no modified nucleotide is used is also one of preferable embodiments of the present invention.

The nucleic acid of the present invention can be produced by chemically synthesizing the passenger strand sequence completely complementary to the guide strand sequence (and the additional sequence (CC)) and 3' overhang (UU) based on the sequence information on the guide strand of naturally occurring miR-29b, ligating a proline derivative linker to its 5'-end by the method described in WO 2012/017919, and then chemically synthesizing the guide strand sequence and the additional sequence in the direction of 3' → 5' from the 5' -end of the passenger strand sequence.

### 2. The pharmaceutical of the present invention

Since miR-29b targets mRNAs encoding factors involved in angiogenesis and the components of extracellular matrixes and has a function that suppresses the expression of these factors, a pharmaceutical comprising the nucleic acid of the present invention as an active ingredient is useful to suppress corneal angiogenesis and scarring and treat corneal diseases (ocular surface disorders and the like). The corneas diseases include, but are not limited to, refractory ocular surface diseases such as Stevens-Johnson syndrome, ocular cicatricial pemphigoid, thermal and chemical burns and the like, various corneal inflammations, hypoxia due to contact lens, infectious corneal diseases, corneal opacity due to reduced function of corneal endothelium and gelatinous drop-like corneal dystrophy. In one embodiment, a pharmaceutical comprising the nucleic acid of the present invention as an active ingredient can be preferably used for treating Stevens-Johnson syndrome, ocular cicatricial pemphigoid, thermal and chemical burns and gelatinous drop-like corneal dystrophy.

The pharmaceutical of the present invention can comprise, in addition to an effective amount of the nucleic acid of the present invention, any carrier, for example, a pharmaceutically acceptable carrier, and applied as a pharmaceutical in the form of a pharmaceutical composition.

Examples of the pharmaceutically acceptable carrier include excipients such as sucrose, starch and the like, binders such as cellulose, methylcellulose and the like, disintegrants such as starch, carboxymethylcellulose and the like, lubricants such as magnesium stearate, aerogel and the like, aromatics such as citric acid, menthol and the like, preservatives such as sodium benzoate, sodium bisulfite and the like, stabilizers such as citric acid, sodium citrate and the like, suspending agents such as methylcellulose, polyvinyl pyrrolidone and the like, dispersing agents such as surfactant and the like, diluents such as water, saline and the like, base wax and the like.

To facilitate the introduction of the nucleic acid of the present invention into a corneal cell, the pharmaceutical of the present invention can further comprise a reagent for nucleic acid introduction. Cationic lipids such as atelocollagen; liposome; nanoparticle; Lipofectin, Lipofectamine, DOGS (Transfectam), DOPE, DOTAP, DDAB, DHDEAB, HDEAB, polybrene, or poly(ethyleneimine) (PEI) and the like, and the like can be used as the reagent for nucleic acid introduction.

Preferably, the pharmaceutical of the present invention is a pharmaceutical composition wherein the nucleic acid of the present invention is encapsulated in a liposome. A liposome is a microscopic closed vesicle having an inner phase enclosed by one or more lipid bilayers, and typically can retain a water-soluble substance in the inner phase and a lipophilic substance in the lipid bilayer. Herein, when the term "encapsulated" is used, the nucleic acid of the present invention may be retained in the inner phase of the liposome or in the lipid bilayer. The liposome to be used in the present invention may be a single layer membrane or a multi-layer membrane. The particle size of the liposome can be appropriately selected within the range of, for example, 10 - 1000 nm, preferably 50 - 300 nm. Considering the delivery efficiency to a corneal tissue, the particle size can be, for example, 200 nm or less, preferably 100 nm or less.

Methods of encapsulating a water-soluble compound such as nucleic acid into a liposome include lipid film method (vortex method), reversed-phase evaporation method, surfactant removal method, freeze-thawing method, remote loading method and the like, but are not limited thereto, and any known method can be appropriately selected.

The pharmaceutical of the present invention can be locally administered into eyes of a mammal. In particular, it is desirable to be instilled into eyes.

Preparations suitable for ocular topical administration include an eye drop (aqueous eye drop, non-aqueous eye drop, emulsion eye drop etc.), ointment, lotion, cream and the like. When the agent of the present invention is an eye drop, a base can be appropriately used. The bases to be used for eye drop include phosphate buffer, Hank's buffer, saline, perfusion fluid, artificial lacrimal fluid and the like.

When the pharmaceutical of the present invention is a preparation for ocular topical administration, for example, buffering agent, isotonicity agent, solubilizing agent, preservative, viscosity base, chelating agent, algefacient, pH adjuster, antioxidant and the like can be selected and added as appropriate.

Examples of the buffering agent include phosphate buffering agent, borate buffering agent, citrate buffering agent, tartrate buffering agent, acetate buffering agent, amino acid and the like.

Examples of the isotonicity agent include saccharides such as sorbitol, glucose, mannitol and the like, polyvalent alcohols such as glycerol, propylene glycol and the like, salts such as sodium chloride and the like, boric acid and the like.

Examples of the solubilizing agent include non-ionic surfactants such as sorbitan polyoxyethylene monooleate (e.g., polysorbate80), polyoxyethylene hydrogenated castor oil, Tyloxapol, pluronic and the like, polyvalent alcohols such as glycerol, macrogol and the like, and the like.

Examples of the preservative include quaternary ammonium salts such as benzalkonium chloride, benzethonium chloride, cetyl pyridinium chloride and the like, paraoxybenzoates such as methyl p-hydroxybenzoate, ethyl parahydroxybenzoate, propyl p-hydroxybenzoate, butyl p-hydroxybenzoate and the like, benzyl alcohol, sorbic acid and a salt thereof (sodium salt, potassium salt and the like), thimerosal (trade name), chlorobutanol, sodium dehydroacetate and the like.

Examples of the viscosity base include water-soluble polymers such as polyvinylpyrrolidone, polyethylene glycol, poly(vinyl alcohol) and the like, celluloses such as hydroxyethylcellulose, methylcellulose, hydroxypropylmethylcellulose, sodium carboxymethylcellulose and the like, and the like.

Examples of the chelating agent include sodium edetate, citric acid and the like.

Examples of the algefacient include *l*-menthol, borneol, camphor, eucalyptus oil and the like.

Examples of the pH adjuster include sodium hydroxide, potassium hydroxide, sodium carbonate, sodium hydrogen carbonate, boric acid or a salt thereof (borax), hydrochloric acid, citric acid or a salt thereof (sodium citrate, sodium dihydrogen citrate etc.), phosphoric acid or a salt thereof (disodium hydrogen phosphate, potassium dihydrogen phosphate etc.), acetic acid or a salt thereof (sodium acetate, ammonium acetate etc.), tartaric acid or a salt thereof (sodium tartrate etc.) and the like.

Examples of the antioxidant include sodium bisulfite, dried sodium sulfite, sodium pyrrosulfite, mixed tocopherols concentrate and the like.

The content of the nucleic acid of the present invention in the pharmaceutical composition of the present invention is, for example, about 0.1 - 100 wt% of the total pharmaceutical composition.

When the pharmaceutical composition of the present invention is a liposome preparation, the molar ratio of the nucleic acid of the present invention to liposome constituents is generally 1/100,000 - 1/10,000. The content of the liposome encapsulating the nucleic acid of the present invention contained in the liposome preparation is not particularly limited, as long as it is an amount in which liposome particles do not aggregate and sufficient efficacy can be exerted, and generally 10 - 100 mM.

The dose of the pharmaceutical of the present invention varies depending on the object of administration, method of administration, kind of ocular surface disorder, size of lesion, situation of the subject of administration (sex, age, body weight and the like). When the pharmaceutical of the present invention is instilled into eyes of an adult human, it is preferable to administer generally 0.01 - 1000 µg, preferably 0.05 - 100 µg, more preferably 0.1 - 50 µg, as a single dose of the nucleic acid of the present invention once to 10 times, preferably 5 - 10 times, per day.

### [Example]

While the Examples of present invention are explained in the following, the present invention should not be limited by these Examples.

### Example 1 Preparation of single stranded artificially matched miR-29b

Based on the sequence information on the 3' side mature miRNA sequence (mmu-mir-29b-3p; miRBase Accession No. MIMAT0000127) derived from the pre-miRNA of naturally occurring mouse miR-29b1 (mmu-mir-29-1; miRBase Accession No. MI0000143), an oligo RNA consisting of the guide strand sequence (underlined) and an additional sequence (CC) (SEQ ID NO:2) was designed, an oligo RNA consisting of the sequence completely complementary thereto and 3' overhang (UU) (SEQ ID NO:3) was synthesized according to a conventional method, and purified by HPLC.
5'-UAGCACCAUUUGAAAUCAGUGUUCC-3' (SEQ ID NO:2)
5'-GGAACACUGAUUUCAAAUGGUGCUAUU-3' (SEQ ID NO:3)

Next, according to the method described in WO 2012/017919, a proline derivative linker represented by the following formula (Ia): 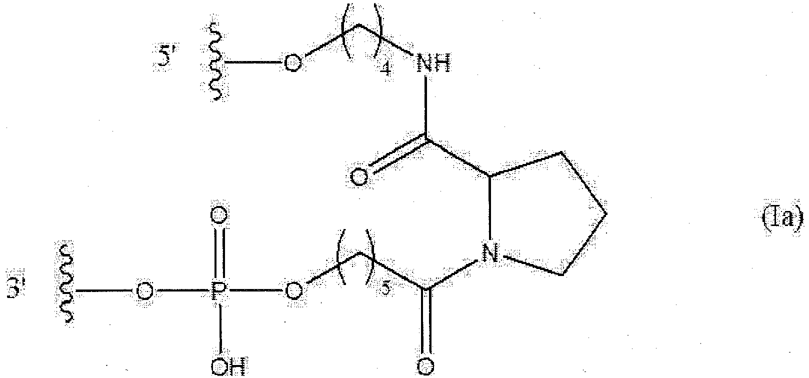 was linked to the 5'-end of the RNA of SEQ ID NO:3. Furthermore, the RNA of SEQ ID NO:2 was synthesized from the 5'-end of the RNA of SEQ ID NO:3 via the linker. The thus-obtained RNA molecule (hereinafter to be referred to as miR29b-PshRNA in Examples) forms the following double stranded structure by self-annealing. 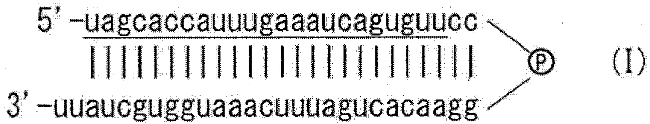 (The guide strand sequence is underlined. P shows the above-mentioned proline derivative linker.)

In the same manner, a single stranded RNA wherein a non-specific guide strand sequence and a sequence completely complementary thereto are linked via the above-mentioned proline derivative linker (SEQ ID NO:4) was synthesized as a negative control.
5'-uacuauucgacacgcgaaguucc-P-ggaacuucgcgugucgaauaguauu-3' (SEQ ID NO:4)

### Example 2 Effects of single stranded artificially matched miR-29b on gene expression in cultured human corneal parenchymal cells

Primary culture of human corneal parenchymal cells was cultured in a 10 cm petri dish using a culture solution consisting of Dulbecco's modified Eagle medium (DMEM)/F12 medium (Sigma Aldrich, USA) supplemented with 10% fetal bovine serum (FBS). The primary culture of human corneal parenchymal cells up to 3 - 5 passages was seeded onto a 24-well plate at 40,000 cells/well. When the cells reached 70-80% confluent, the miR29b-PshRNA or negative control RNA prepared in Example 1 (the final concentration of each RNA was 25 nM) was introduced into the cells using Lipofectaimin RNAiMAx® (Invitrogen) according to the manufacturer's protocol. After 48 hrs, the cells were collected, total RNA was extracted, and the expression levels of TGF-β, Col1A1, VEGF and Angpt12 genes were quantified by real time RT-PCR. The results are shown in Fig. 1-1 and Fig. 1-2. The expression of angiogenesis-promoting factors (TGF-β, VEGF and Angptl2) and collagen (Col1A1) in the cultured human corneal parenchymal cells was suppressed by the addition of the miR29b-PshRNA.

These results suggest the possibility that miR-29b replacement therapy is useful to treat corneal angiogenesis and cicatricial opacity.

### Example 3 Treatment effect of instillation of miR29b-PshRNA liposome preparation on mouse alkaline burn model

Accordingly, the treatment effect of the miR29b-PshRNA was confirmed using a mouse alkaline burn model. To improve introduction efficiency of the miR29b-PshRNA into corneal cells, the RNA was administered in the form of liposome encapsulating same.

### (1) Preparation of liposome preparation

The preparation of miR29b-PshRNA-encapsulated liposomes was performed by thin film hydration method. Distearoyl phosphatidylcholine (DSPC), cholesterol and stearylamine were weighed such that their molar ratio is adjusted to 7:3:1 in an eggplant flask, and dissolved in an adequate amount of chloroform. The solvent was evaporated under reduced pressure using a rotary evaporator in water bath at 40°C to prepare a thin film. The thin film was dried under reduced pressure overnight, hydrated using 1 µM miR29b-PshRNA solution (in 1 mM TE buffer; pH 7.4) in water bath at 70°C, and incubated at room temperature for 15 min to prepare miR29b-PshRNA encapsulated multi-layer membrane liposomes (MLVs). The obtained MLVs were passed through a filter having a pore size of 100 nm 41 times using an extruder (LiposoFast™-Pneumatic, AVESTIN) under the pressure of 150-200KPa to prepare liposomes miniaturized into submicron size (mir29b liposome-PshRNA) (DSPC concentration is 20 mM in the liposome preparation). As a control, liposomes encapsulating a non-specific PshRNA (non-specific liposome-PshRNA) were prepared in the same manner.

### (2) Administration to mouse alkaline burn model by ocular instillation

Five microlitters of 0.5 N NaOH was instilled into eyes of 6-8-week-old C57BL/6 mice (day 0), and rinsed away with 20 ml of PBS after 30 seconds. Then, the mir29b liposome-PshRNA or non-specific liposome-PshRNA (5 µl) prepared in (1) above was ocularly instilled (PBS was ocularly instilled as a control), and wiped off with KimWipe™ (Nikkei Products Co. Osaka, Japan) after 5 minutes. Then, Ofloxacin eye ointment (Talibit®, Santen, Japan) was instilled. The instillation was performed every 24 hours and continued until Day 9.

On Day 10, ketamine hydrochloride (35 mg/kg) and xylazinechloride (5 mg/kg) were administered to the mice. After confirming the death of the mice sacrificed by cervical dislocation, eye balls were excised and corneal mounts were prepared.

### (blood vessel quantification)

The cornea was fixed with acetone at -20°C and rinsed with PBS 3 times. The corneal section was fixed with 1% bovine serum albumin (Sigma-Aldrich) and 0.5% Triton (Sigma-Aldrich) dissolved in PBS (fixative) at room temperature for 48 hours, and immersed in a solution containing rat anti-mouse CD31 antibody (BD Biosciences, Franklin Lakes, NJ) diluted 1:500 with the fixative and stained at 4°C overnight. After washing with PBS, the corneal section was immersed in a solution containing the secondary antibody (Alexa Fluor 594-labelled donkey anti-rat IgG; Invitrogen, San Diego, CA) diluted 1:1000 with the fixative and stained at room temperature for 5 hours. A mount was prepared by VECTASHIELD® mounting medium (Vector Laboratories, CA, USA), and shot with a fluorescence microscope (BZ-9000; Keyence, Osaka, Japan). Blood vessel region was measured using NIH Image software (Image J; http://rsb.info.nih. gov/ij/), percentage of the blood vessel area per unit corneal area was calculated and comparatively reviewed. The results are shown in Fig. 2. The administration of mir29b liposome-PshRNA by ocular instillation suppressed angiogenesis and scarring in alkaline burn.

These results demonstrate that miR-29b replacement therapy by ocular instillation is useful as a novel therapeutic method for refractory ocular surface disorders accompanied by corneal angiogenesis and cicatricial opacity.

### [Industrial Applicability]

A pharmaceutical comprising the nucleic acid of the present invention as an active ingredient is useful to treat corneal diseases such as ocular surface disorders and the like, particularly refractory ocular surface disorders accompanied by corneal angiogenesis and cicatricial opacity.

This application is based on JP 2015-085470 filed on April 17, 2015 in Japan, which is incorporated by reference herein in its entirety.

## Claims

1. A single stranded nucleic acid molecule represented by the following formula (I):
5'-uagcaccauuugaaaucaguguucc-P-ggaacacugauuucaaauggugcuauu-3' (I)
(SEQ ID NO:1),
wherein -P- shows a proline derivative linker represented by the following formula (Ia):

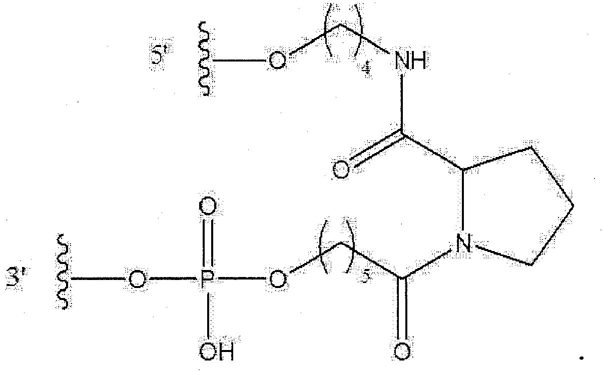

2. An agent for treating a corneal disease comprising the single stranded nucleic acid molecule according to claim 1 as an active ingredient.

3. The agent according to claim 2, wherein the corneal disease is accompanied by corneal angiogenesis or cicatricial opacity.

4. The agent according to claim 3, wherein the corneal disease is selected from the group consisting of an ocular surface disorder, corneal inflammation, hypoxia due to contact lens, an infectious corneal disease, corneal opacity due to reduced function of corneal endothelium and gelatinous drop-like corneal dystrophy.

5. The agent according to claim 3, wherein the corneal disease is a refractory ocular surface disorder selected from the group consisting of Stevens-Johnson syndrome, ocular cicatricial pemphigoid, and thermal and chemical burns.

6. The agent according to any one of claims 2 to 5, wherein the single stranded nucleic acid molecule is encapsulated in a liposome.

7. The agent according to any one of claims 2 to 6, which is an eye drop.

8. A method of treating a corneal disease, comprising administering to a subject in need thereof a therapeutically effective amount of the single stranded nucleic acid molecule according to claim 1.

9. The stranded nucleic acid molecule according to claim 1 for use in the treatment of a corneal disease.

10. Use of the single stranded nucleic acid molecule according to claim 1 for the manufacture of an agent for treating a corneal disease.
